# EUROPEAN PATENT APPLICATION

(11) **EP 1 635 178 A2**
(43) Date of publication of application: **15.03.2006**
(21) Application number: 04021801.8
(22) Date of filing: 14.09.2004
(51) Int. Cl.: G01N 33/68

(54) **Breast cancer proteins**

(71) Applicant: ProteoSys AG, 55129 Mainz (DE)
(72) Inventor: Cahill, Michael, 55296 Loerzweiler (DE); Schrattenholz, André, 55129 Mainz (DE)
(74) Representative: Patentanwälte Ruff, Wilhelm, Beier, Dauster & Partner

(57) **Abstract**

The present invention relates to a method for performing proteomics especially with human microdissected samples based on a pooling strategy that groups the samples into two or more pools based on parameters of interest, said pools revealing at least one protein that is differentially manifest between these pools but common to the members of a given pool.

## Description

### Introduction

There is a need to improve the definition of molecular events underlying cancer to develop improved diagnostic and therapeutic avenues. Model systems, such as cell culture models, are often employed in an attempt to achieve this. However much doubt exists as to how well results from such systems reflect the biology that is relevant in human disease. Therefore it is highly desirable to examine cells from primary clinical tissues. Gene array studies are commonly used because of the availability of suitable standard protocols. However protein-based phenomena, such as altered turnover or post-translational modifications, are not detected by gene arrays.

Proteomics is the study of proteins in complex biological systems. Current proteomics methods are poorly suited to examination of most primary human samples, because the amounts of material that can be harvested are often extremely limited. Furthermore, the relevant disease cells are often mixed with many other cell types in tissues, requiring application of microdissection techniques which exacerbate the sample yield problem.

When extremely limited protein amounts are measured with poor sensitivity the estimate of protein abundance is determined with poor certainty. Thus numerous replicates are required to achieve statistically robust results, which is seldom possible with rare samples. Therefore high sensitivity methods are extremely advantageous for the quantification of limited amounts of protein. Actually, there are relatively few proteomics results published on clinical tumours, and statistically robust differential data are accordingly scarce. This broad problematic is exacerbated by modern screening programs, which detect tumours at an earlier and smaller stage.

We examined breast cancer as a model system to develop improved proteomics methods based upon ProteoTope differential display (Cahill et al., 2003). We observed that the limit of sensitivity involves the step of radiolabelling (data not shown). In order to reconcile the amounts of protein obtainable from microdissection experiments on primary human samples with the amounts of protein required for high quality results, we devised a sample pooling strategy.

Briefly, samples are subjectively grouped into two or more pools of interest based on parameters of interest, and consistent differences between these pools reveal traits which are more or less universally differentially manifest between the pools, but more or less common to members of a given pool. Variables whose abundances differ independently of pool affiliation should not generate consistently significant differences when pools are compared, and are essentially ignored.

There are two main situations where pooling is advantageous. The first is to reduce the costs of screening large numbers of samples. The second is where experimental restraints make measurements of individual samples difficult or impossible. Although the benefits of sample pooling have been considered in other areas (e.g. Kendziorski et al., 2003) to our knowledge, sample pooling has not been utilised in proteomics. For the proteomics analysis of microdissected clinical cancer samples, both situations apply.

This object has been solved by the method according to claim 1. Further embodiments of the invention are depicted in the dependent claims 2-10. The invention further comprises the use of said method for performing proteomics as claimed in claim 11 and of several proteins as diagnostic markers as claimed in claims 12-26. The wording of all claims is hereby made to the content of the specification by reference.

In this work we devised and tested a pooling strategy using large homogenous invasive ductal breast carcinomas, which are well suited for conventional proteomics analysis, yet are uncommon and becoming increasingly rare. This test study involved pools that were positive or negative for the estrogen receptor. We then applied the validated pooling approach to a set of more typical heterogenous invasive tumours, all of which were ER+, and examined pools positive or negative for the progesterone receptor. Because of the microheterogenous nature of these tumours, a study without microdissection would not have been appropriate. Also, because only small fractions of each tissue contained tumour cells, the study would not have been possible on individual tumours without sample pooling.

Breast cancer is one of the most common forms of cancer observed in women, with a predicted number of approx. 215 990 (32%) new cases and with approx. 40 110 deaths in the US in 2004 (American Cancer Society, 2004; Jemal et al., 2004). In Germany breast cancer has the highest incidence rate and mortality among women (1997: 45 800 and 18 378, respectively. Bray et al., 2001; Sauer, 2001). Endogenous estrogens, which have effects on many organs, are thought to play a major role in the development of the breast, suggesting that an increased sensitivity or longer exposures to estrogens is involved in a higher risk of tumorigenesis (Fisher et al., 2001; Osborne, 1998; Henderson, 1993).

Estrogen effects are exerted through two types of specific nuclear receptors - estrogen receptor alpha (ERα) and beta (ERβ) - inducing ligand-dependent transcription (Platet et al., 2004). Molecular analysis has shown that ERα, like other nuclear receptors, consists of separable domains responsible for DNA binding [DNA-binding domain (DBD), hormone binding domain (HBD)], and transcriptional activation. The N-terminal activation function (AF-1) of the purified receptor is constitutively active, whereas the activation function located within the C-terminal part (AF-2) requires hormone for its activity (Platet et al., 2000).

ERα is found in 50-80% of breast tumors and ERα status is essential in making decisions about endocrine therapy with anti-estrogens, which are competitive inhibitors of endogenous estrogens and inhibit the mitogenic activity of estrogens in breast cancer. On a molecular basis, they trigger inactive conformation of the ERα, which is then unable to activate transcription (Shiau et al., 1998). The anti-estrogen tamoxifen is used to treat hormone-dependent breast cancers as a systemic adjuvant therapy after surgery for early breast cancer, where, after 5 years of treatment, it reduces disease recurrence and improves survival regardless of patient age and nodal status (Come et al., 2003). In patients with metastatic disease who have ER-positive tumors, tamoxifen is effective in approximately 50% of the cases (Fisher et al., 2001).

While the mitogenic action of estrogens in breast cancer cells is well established, there is also evidence that estrogen receptors mediate protective, anti-invasive effects. Clinically, a positive ER status correlates with favourable prognostic features, including a lower rate of cell proliferation and histologic evidence of tumor differentiation. ER status is also prognostic for the site of gross metastatic spread. For reasons unknown, ER+ tumors are more likely to initially manifest clinically apparent metastases in bone, soft tissue, or the reproductive and genital tracts, whereas ER- tumors more commonly metastasise to brain and liver. Several studies have correlated ERα expression to lower Matrigel invasiveness and reduced metastatic potential of breast cancer cell lines (Platet et al., 1998; Thompson et al., 1992).

Moreover, when ERα -positive cells are implanted in nude mice, tumors appear only in the presence of estrogens and are poorly metastatic as compared to those developed from ERα -negative breast cancer cell lines (Price et al., 1990). This paradox suggests that ERα expression could be associated with or involved in pathways that hinder cancer progression. Therefore, downstream molecular targets should be identified as possible targets to modify such pathways and to investigate the mechanism of this effect, which could be due to modulation of estrogen-specific genes involved in metastasis or to transcriptional interference of other proteins by squelching of transcription factors or competition on promoter sequences, for example. During the course of this work, valuable discoveries were made.

### Materials and Methods

### Asservation of tumor samples

After removal of breast tumor from the patient, samples are characterized and collected by an experienced pathologist, snap frozen in liquid nitrogen in between 15 minutes after tumor removal and stored at -196 degree Celsius in a tumor tissue bank. Sample collection is approved by an ethics committee and by the patient. Tumor data are stored in an Oracle-based database (IEEE-Standard).

### Preparation of cryosections

Tumor samples are selected using the database, removed from the tissuebank on dry ice and transfered to a cryotom (Leica) at a temperature of -23 °C. The tissue samples are embedded in OCT (Leica) and snap frozen in liquid nitrogen. Then sections (6 µm) are made, placed on SuperFrost+-slides (Multimed) and stored at - 80°C until further use. For immunopathologic characterisation by an experienced pathologist one section is stained with hematoxilin/eosin and tumor cells for laser microdissection are identified. The quality of each tumour was verified by measuring RNA integrity on an Agilent 2001 Bioanalyser. Tumours with degraded RNA were excluded from the study.

### Laser microdissection

Using a laser capture micricope (Arcturus-PixCell-Ile) tumor cells were isolated, transfered into tubes, stored at - 80°C, and transported on dry ice, according to manufacturer's instructions.

### Sample preparation

Protein Alkylation, iodination, 2D-PAGE and data analysis were according to a variation of Vuong et al. (2000), Cahill et al. (2003), and Sommer et al. (2004). Radioactive iodine was purchased from Amersham Biosciences (Freiburg). Protein iodination reactions with either I-125 or I-131 were conducted separately with identical chemical iodine concentrations as described (Cahill et al., 2003).

### Two Dimensional ProteoTope analysis

2D-PAGE was performed using 18 cm commercial IPGs of pH 4-7 (Amersham) and 20 cm SDS-PAGE gels (Hoefer ISO-DALT), or own construction pH 4-9 IPGs of 54 cm that were run in the SDS-PAGE dimension as 3 x 18cm IPGs in an ISO-DALT as described (Poland et al., 2003). The ProteoSys proprietary ProteoTope platform (Cahill et al., 2003) is a combination of methods developed by ProteoSys AG, including radio-iodination, 2D-PAGE, and high sensitivity radio-imaging. ProteoTope includes proprietary radio-imaging, which can discriminate between ¹²⁵I and ¹³¹I signals in one 2D-PAGE gel to generate a quantitative multicolour differential display of proteins from separate samples labelled with different iodine isotopes.

ProteoTope gels are produced for quantification, since the ProteoTope technology allows the most accurate quantification. Because the signal is radioactive, error is proportional to the square root of the number of detected signals. For all but the weakest signals, the measurement errors are negligible for subsequent differential quantification. No image registration (spot alignment) of the two sample images acquired from one gel is necessary. Thus a direct comparison of integrated spot intensities for the samples run on one gel can be used for further analysis. A synthetic reference must be introduced in order to compare the image pairs in gel 1 with those on gel 2 (see Figure 1).

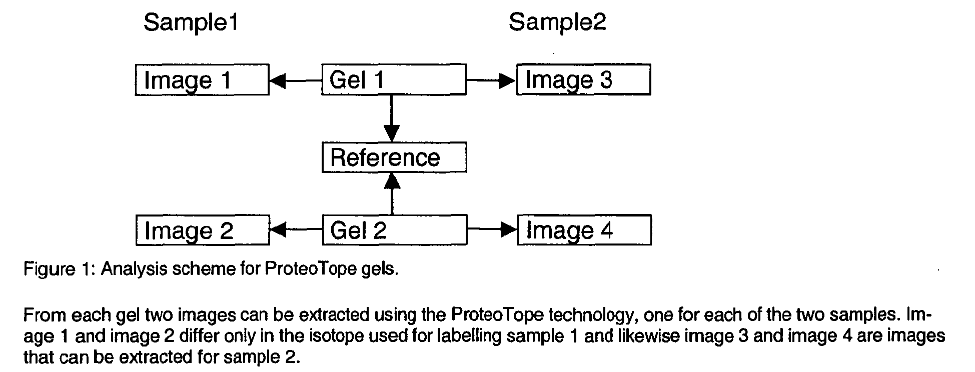

Since for ProteoTope gels the two samples to compare are run on one gel the expression analysis does not suffer from inter gel variations as silver stained gel analyses do. The major source of error is a potential bias towards the labelling stoichiometry of one or the other isotope. This can be monitored by producing the gels in inverse matched cross labelled duplicate fashion. When the images of one sample labelled with each of two isotopes do not correspond, quality criteria are not met and the analysis is repeated.
For ProteoTope gels a dual colour overlay can be generated with some computational effort to highlight differences between the two samples. Consistent differences between the samples will appear in both images in alternate channels.

### Tracer Control Enrichment Gels

Analytical two colour ProteoTope gels are loaded with too little sample (<1 µg protein) to permit protein identifications with current methods. For preparative protein identification, tracer control enrichment gels (tracer gels) are employed, where a trace of radioactively labelled protein sample, corresponding to the sample used for analytical two colour gels, is co-electrophoresed with a vast excess of non-radioactively labelled protein (about 200 µg) to provide preparative amounts of protein for identification. Overlaying the silver stained gel images and the ProteoTope radioactive gel images permits us to locate the position of ProteoTope-detected proteins on gels that are silver stained for spot picking. These images are not used for quantification. On each gel 200 µg of protein is applied and electrophoresis is performed as described before for ProteoTope gels (Vogt et al., 2003; Cahill et al., 2003). After electrophoresis gels are silver stained according to Shevchenko et al. (1996).

### Image analysis

The differential protein expression analysis is based on the reliable differential quantification of the protein spots on ProteoTope gels. For the quantitative image analysis we employ the Phoretix 2D Advanced software (Nonlinear Dynamics), Delta 2D (De-codon GmbH) and GREG (Fraunhofer-Institut für Angewandte Informationstechnik). ProteoSys has added tools for automated data extraction, statistical analysis, and report generation.

### Image Quantification Protocol

The analysis of ProteoTope and silver stained gels is typically performed according to the following protocol (e.g. for Phoretix 2D):

### Detecting spots for each image

- Calibration of image intensity to counts (for ProteoTope images only)
- Automatic spot detection with manual adjustment of detection parameters (for Phoretix 2D these parameters are called sensitivity, noise, operator size, and background)
- Manual splitting or merging of spots when needed
- Background subtraction with mode of non-spot which uses a number of pixels around each spot that are not part of any other spot to determine the background

### Matching the images against a reference gel

- Manually defining user seeds to give an initial set of matching spots
- Automatic matching of all spots in all images against the reference gel
- Manual correction to unmatched spots if needed

### Creating a table of spots of special interest

- Normalizing the spot intensities to the reference using the match ratio optimisation
- Highlighting spots that are up- or down regulated by more than 33 % to facilitate manual screening
- Manual selection of spots that should be further analysed

### Generating a spot report

- Exporting the complete analysis
- In house developed software is used to extract a report that shows the selected spots together with the quantification results and statistics

### Spot Selection

Spot selection is based on quantitative criteria as well as gel image quality and spot detection quality. For statistical calculations the logarithm to base 2 of the intensity ratio of the two samples applied on the gel is used i.e. log₂(Iₛₐₘₚₗₑ₁/Iₛₐₘₚₗₑ₂). The resulting quantity is usually called logodd. The mean values and standard errors are modeled from the logodd values taking advantage of the gel replicates and using the patients as random effect. This allows calculation of p-values using a t-test that is based on the model. If there is good evidence from the gel images that one of the spot intensities is an outlier, e.g. stripes or other distortions occur, then this one spot's intensity may be discarded. If a spot is not detected on one image its intensity is set to zero for that image unless it can clearly be identified as an outlier, in which case it will be discarded.

In general a spot is selected for further analysis if the p-value is lower than 1% and the absolute logodd value is higher than 0.6 for ProteoTope, corresponding to a ratio of 1.5. At that point the image quality is checked to assure that the spot was consistently detected on all images. Additional spots may be selected even if the p-value is less higher than 1%: e.g. if the expression ratio is high, if the spot is in the close vicinity of a significantly different spot, or if there are no or very few spots that are significantly different otherwise. In any case the table with the quantitative analysis is presented without modifications to allow us to estimate how reliable a difference is from the statistical point of view. The ProteoTope inverse labelled cross replicate data structure resembles that of replicated microarray experiments, for which established statistical methods are available (Pan, 2002).

### Protein Identification by Mass Spectrometry

Protein identification is based on different mass spectrometric methods: an automated procedure that allows a very quick and reliable identification of higher abundant proteins (peptide mass fiungerprinting with MALDI-TOF-MS) but also allows the identification of very low abundant proteins with more time consuming procedures (LC-ESI-IonTrap-MS/MS, or MALDI TOF-TOF). Briefly, gel plugs of selected protein spots are excised and the proteins contained in the gel plugs are digested using trypsin. The resulting solution is analysed first with a high throughput peptide mass fingerprint procedure based on MALDI-TOF-MS. For those spots where only ambiguous identification was achieved, a fragment ion analysis based on MALDI TOF-TOF or LC-ESI-IonTrap-MS/MS was added. A detailed description of typical MALDI-TOF-MS procedures has been published (Vogt et al. 2003).

### Database Searching

For the identification of the proteins the peptide masses extracted from the mass spectra were searched against the NCBI non-redundant protein database (www.ncbi.nlm.nih.gov) using MASCOT software version 1.9 (Matrix Science, London, detailed description can be found at http://www.matrixscience.com/).

The described features of the invention and further features result in greater detail from the examples in combination with the subclaims. The features could be realized in combination with each other or alone.

### Experiment 1: ER positive vs ER negative

### (Whole tumour Slices)

In this project we needed to develop pooling strategies to enable ProteoTope analysis of microdissected protein samples. Various pooling strategies are possible. We devised the design discussed below, and first tested its performance using whole tissue sections from large homogenous breast tumours that were pooled on the basis being either positive (+) or negative (-) for the estrogen receptor (ER).
8 ER+ tumours and 8 ER- tumours were used for this pilot study. They were randomly assigned to sub-pools, each containing normalised equal amounts of protein from two tumours (Table 1).

**Table 1. Pooling design for experiment 1. ER+ vs ER-, cryogenic tissue sections.**

| ER+ pools | T378 | T392 | T460 | T464 | T288 | T711 | T712 | T425 |
|---|---|---|---|---|---|---|---|---|
| ER+1 | + | + | | | | | | |
| ER+2 | | | + | + | | | | |
| ER+3 | | | | | + | + | | |
| ER+4 | | | | | | | + | + |
| | | | | | | | | |

| ER- pools | T433 | T443 | T469 | T470 | T531 | T558 | T623 | T640 |
|---|---|---|---|---|---|---|---|---|
| ER-1 | + | + | | | | | | |
| ER-2 | | | + | + | | | | |
| ER-3 | | | | | + | + | | |
| ER-4 | | | | | | | + | + |

Individual tumours are designated by their tumour bank registration numbers.

For differential analysis, sub-pool ER+1 (containing T378 and T392) was differentially compared to sub-pool ER-1 (containing T433 and T443), ER+2 was compared to ER-2, ER+3 was compared to ER-3, and ER+4 was compared to ER-4. The statistically most significant differential protein spots are presented in Table 2, and details of their identification in Table 3.

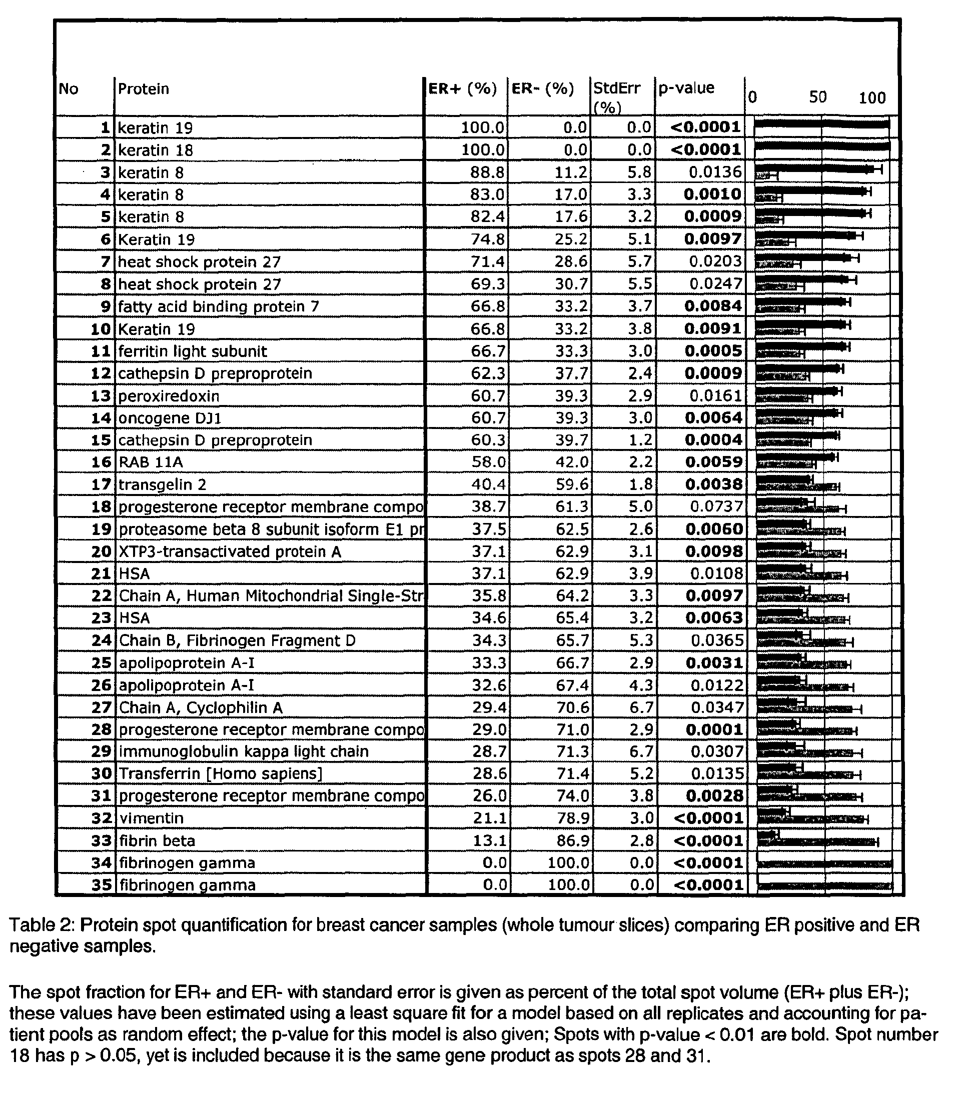

**Table 3. Details of identification of proteins from Table 2. No= Spot Number from Table 2. PI= isoelectric point. MW= molecular weight. Exp.= experimentally observed values (of PI & MW). AccNo= ncbi (National Center for Biotechnology Information) Accession Number.**

| **Spot No** | **exp. PI** | **MW** | **PMF Score** | **AccNo** | **Description** |
|---|---|---|---|---|---|
| 01 | 4.9 | 41000 | 204 | gi\|34783124 | Keratin 19 [Homo sapiens] |
| 02 | 5.3 | 45000 | 80 | gi\|4557888 | keratin 18; cytokeratin 18 [Homo sapiens] |
| 05 | 5.4 | 52000 | 218 | gi\|4504919 | keratin 8; cytokeratin 8; keratin, type II cytoskeletal 8 |
| 04 | 5.4 | 55000 | 419 | gi\|4504919 | keratin 8; cytokeratin 8; keratin, type II cytoskeletal 8 |
| 05 | 5.5 | 51000 | 214 | gi\|4504919 | keratin 8; cytokeratin 8; keratin, type II cytoskeletal 8 |
| 06 | 5.0 | 42000 | 395 | gi\|34783124 | Keratin 19 [Homo sapiens] |
| 07 | 5.8 | 26000 | 173 | gi\|662841 | heat shock protein 27 [Homo sapiens] |
| 08 | 5.5 | 26000 | 125 | gi\|662841 | heat shock protein 27 [Homo sapiens] |
| 09 | 5.2 | 13000 | 116 | gi\|4557585 | fatty acid binding protein 7, brain; mammary-derived growth inhibitor-related [Homo sapiens] |
| 10 | 5.0 | 43000 | 407 | gi\|34783124 | Keratin 19 [Homo sapiens] |
| 11 | 5.4 | 19000 | 113 | gi\|20149498 | ferritin, light polypeptide; ferritin light polypeptide-like 3 |
| 12 | 5.2 | 29000 | 98 | gi\|4503143 | cathepsin D preproprotein [Homo sapiens] |
| 13 | 5.4 | 21000 | 151 | gi\|33188452 | peroxiredoxin 2 isoform b; thioredoxin-dependent peroxide reductase 1; thiol-specific antioxidant 1; natural killer-enhancing factor B; thioredoxin peroxidase 1; torin |
| 14 | 5.9 | 23000 | 115 | gi\|31543380 | RNA-binding protein regulatory subunit; oncogene DJ1 |
| 15 | 5.5 | 29000 | 76 | gi\|4503143 | cathepsin D preproprotein [Homo sapiens] |
| 16 | 5.8 | 24000 | 109 | gi\|4758984 | Ras-related protein Rab-11A; RAB 11A, |
| 17 | 7.4 | 20000 | 139 | gi\|4507357 | transgelin 2; SM22-alpha homolog [Homo sapiens] |
| 18 | 4.5 | 22000 | 107 | gi\|5729875 | progesterone receptor membrane component 1 |
| 19 | 7.1 | 23000 | 68 | gi\|4758970 | proteasome beta 8 subunit isoform E1 proprotein; |
| 20 | 4.9 | 19000 | 94 | gi\|1312910 | XTP3-transactivated protein A [Homo sapiens] |
| 21 | 5.6 | 71000 | 144 | gi\|6013427 | serum albumin precursor, human serum albumin |
| 22 | 7.4 | 15000 | 105 | gi\|2624694 | Chain A, Human Mitochondrial Single-Stranded Dna Binding Protein |
| 23 | 5.7 | 71000 | 128 | gi\|6013427 | serum albumin precursor [Homo sapiens] |
| 24 | 5.6 | 40000 | 70 | gi\|2781208 | Chain B, Crystal Structure Of Fibrinogen Fragment D |
| 25 | 5.2 | 24000 | 210 | gi\|4557321 | apolipoprotein A-I precursor [Homo sapiens] |
| 26 | 5.2 | 24000 | 99 | gi\|4557321 | apolipoprotein A-I precursor [Homo sapiens] |
| 27 | 7.0 | 16000 | 85 | gi\|1633054 | Chain A, Cyclophilin A Complexed With Dipeptide Gly-Pro |
| 28 | 4.6 | 22000 | 95 | gi\|5729875 | progesterone receptor membrane component 1; |
| 29 | 7.3 | 25000 | 77 | gi\|21669399 | immunoglobulin kappa light chain VLJ region |
| 30 | 6.0 | 80000 | 99 | gi\|37747855 | Transferrin [Homo sapiens] |
| 31 | 4.5 | 22000 | 110 | gi\|5729875 | progesterone receptor membrane component 1; progesterone binding protein [Homo sapiens] |
| 32 | 4.9 | 45000 | 150 | gi\|4507895 | vimentin [Homo sapiens] |
| 33 | 6.3 | 57000 | 165 | gi\|223002 | fibrin beta |
| 34 | 5.4 | 51000 | 105 | gi\|71827 | fibrinogen gamma-A chain precursor [validated] - human |
| 35 | 5.3 | 49000 | 66 | gi\|71827 | fibrinogen gamma-A chain precursor [validated] - human |

The results observed for this study provided a protein profile that was expected from published studies on this system (Perou et al. 2000; Van't Veer et al., 2002; Gruvberger et al., 2001). We observed extremely elevated levels of keratins 19, 18 and 8 in ER+ tumours, relative to ER- tumours. According to published literature, most ER+ cancers exhibit a phenotype similar to luminal epithelial cells of the milk ductules. Keratins 8 and 18 are characteristic of most secretory cells, while keratins 9 and 19 are present in ductile epithelial cells (Narod and Foulkes, 2004; Chu and Weiss, 2002). Thus our results suggest that ER- tumours may exhibit a less luminal phenotype than ER+ tumours. It is known that ER- tumours exhibit a higher degree of lymphocyte infiltration and inflammatory response than ER+ tumours, as assayed by e.g. higher levels of B-cell and T-cell specific mRNAs (van't Veer et al., 2002). Accordingly, we observe higher amounts of immunoglobulin kappa light chain associated with ER- tumours, and a slight but significant increase in the serum proteins albumin and apolipoprotein A1: possibly representing an oedematous effusion of serous fluid into more voluminous interstitial spaces of lymphocyte infiltrated ER- tumours. Notably, several hemoglobin spots were all slightly more abundant in the ER+ tumour pool with marginal significance (p > 0.05: data not shown), suggesting that ER- tumours may have a lower density of blood vessels.

The most highly differential abundant protein in ER- tumours relative to ER+ tumours in our study was fibrinogen gamma A chain, followed by fibrin. Fibrinogen is proteolytically cleaved under the influence of platelets to produce a fibrin clot during the process of blood clotting. Cancer-related fibrin deposition and fibrinolysis characterises many solid tumours, with cancer cells supplying many of the functions supplied by platelets in normal blood clotting (Gerner et al., 2001). The deposition of fibrinogen without subsequent conversion to fibrin in the tumor stroma is reportedly a hallmark of breast carcinoma. Endogenous synthesis and secretion of fibrinogen is, at least in part, the source of deposition in the extracellular matrix of breast cell carcinomas (Rybarczyk and Simpson-Haidaris, 2000). Estrogen destabilises fibrinogen mRNA in frogs (Pastori et al., 1990; Wangh et al., 1983), and estradiol represses the fibrinogen gamma mRNA in fish (Bowman et al., 2002). Together with our observed downregulation in ER+ tumours, this is suggestive of a possible phylogentically conserved negative effect of estrogen on the abundance of fibrinogen in ER+ cells. Therefore, although fibrinogen/fibrin levels have not to our knowledge been previously associated with ER- status of breast cancers, our observation is consistent with published data. Fibrin is cross linked to form a fibrin gel in a blood clot, however fibrin gels would be neither solubilised by our buffer system, nor would they migrate in 2D-PAGE. Therefore we cannot conclude on more than the observed increase in non-cross linked fibrin in ER- tumours. Indeed since gamma fibrinogen is involved in cross-linking, one possible interpretation is that fibrin gel formation is more pronounced in ER+ tumours. It is also notable that of 5 different spots of fibrinogen beta chain detected, none was significantly different between ER+ and ER- tumours (data not shown). In any case, the biology associated with variability in extracellular matrix of the different tumour types revealed here deserves further examination.

Our study also revealed the presence of different isoforms of several differentially abundant proteins, which differed in the significance of their differential expression (Table 4). This is strongly suggestive of the presence of differential post-translational modifications.

**Table 4. Proteins identified in Table 2 and Table 3 and for which more than one protein spot isoform was identified in 2D-PAGE gels of ER+ vs ER- breast cancer tumours. Each designated spot was observed with different experimental PI and MW. Labelling follows Table 3.**

| **theor** | | **Exp.** | | **AccNo** | **PMF Score** | **Description** |
|---|---|---|---|---|---|---|
| **MW** | **pl** | **PI** | **MW** | | | |
| 45587 | 4.85 | 5.0 | 43000 | gi\|34783124 | 407 | Keratin 19 [Homo sapiens] |
| | | 5.0 | 42000 | | 395 | |
| | | 5.0 | 42000 | | 424 | |
| | | 4.9 | 41000 | | 204 | |
| 55874 | 5.38 | 5.3 | 56000 | gi\|39645331 | 154 | KRT8 protein. keratin 8; cytokeratin 8; keratin, type II cytoskeletal [Homo sapiens] |
| 53671 | 5.26 | 5.1 | 49000 | gi\|4504919 | 102 | 8 |
| | | 5.2 | 49000 | | 215 | |
| | | 5.4 | 55000 | | 419 | |
| 22667 | 8.24 | 5.4 | 26000 | gi\|662841 | 123 | heat shock protein 27 [Homo sapiens] |
| | | 5.5 | 26000 | | 125 | |
| 22638 | 8.96 | 7.8 | 22000 | gi\|4507359 | 95 | transgelin; SM22-alpha [Homo sapiens] |
| | | 8.5 | 21000 | | 137 | |
| 18578 | 7.97 | 6.8 | 16000 | gi\|1633054 | 87 | Chain A, Cyclophilin A |
| | | 7.4 | 15000 | | 81 | |
| | | 7.0 | 16000 | | 85 | |
| 22012 | 4.30 | 4.6 | 22000 | gi\|5729875 | 95 | progesterone receptor membrane component 1; progesterone binding protein; Hpr6.6[Homo sapiens] |
| | | 4.5 | 22000 | | 110 | |
| | | 4.5 | 22000 | | 107 | |
| 53830 | 4.77 | 4.8 | 47000 | gi\|4507895 | 336 | vimentin [Homo sapiens] |
| | | 5.0 | 56000 | | 531 | |
| | | 4.9 | 45000 | | 150 | |
| 58017 | 8.38 | 6.1 | 59000 | gi\|399492 | 79 | Fibrinogen beta chain precursor [Contains: Fibrinopeptide B]. Chain B, Crystal Structure Of Fibrinogen Fragment D |
| | | 6.1 | 57000 | | 85 | |
| | | 6.8 | 55000 | | 66 | |
| | | 7.1 | 57000 | | 88 | |
| 39041 | 6.06 | 5.6 | 40000 | gi\|2781208 | 70 | |

We observed three Vimentin spots in the tumour study, one of which was highly significantly more abundant in ER- tumours. Published literature reports that most vimentin-positive tumours are ER-, and that this correlates with tumour aggressiveness (Niveditha and Bajaj, 2003).

Taken together, the profile of differential protein expression between ER+ and ER- tumours strongly resembled the profile reported from the literature. Thus we were quite confident that the pooling regime indeed was successful at revealing differences associated specifically with the biological states defined by the experimental design (ER+ vs ER- tumours in this case).

With this perspective, it is particularly interesting that we identified three spots corresponding to progesterone receptor membrane component 1, also known as Hpr6.6 protein (Gerdes et al., 1998). Two spots corresponding to this protein were significantly upregulated in ER- tumours (spots 28 and 31 in Table 2). This protein is not related to the classical progesterone receptor. Hpr6.6 has a transmembrane domain N-terminally to a cytochrome b5 domain that does not interact with heme groups, but is rather thought to be involved in steroid binding. Such membrane-associated progesterone receptors are thought to mediate a number of rapid cellular effects not involving changes in gene expression (Mifsud and Bateman, 2002; Lösel et al., 2003). Obviously, they also have the potential to influence gene expression. Hpr6.6 protein regulates the response to oxidative damage in the MCF-7 breast cancer cell line, leading to apoptosis (Hand and Craven, 2003). Our results here are the first to reveal significant differences in Hpr6.6 between ER+ and ER- tumours, and reveal that this protein is potentially a potent target for anticancer therapy of ER- tumours. We also identify that XTP3-transactivated protein A is significantly more abundant in ER- tumours. There is no literature published on this protein in association with cancer. A BLAST search (Altschul et al., 1997) reveals homology with an ATP-binding ABC transporter protein from the prokaryote *Thermotoga maritime* (Figure 2). As such, XTP3-transactivated protein A is also a promising target for anticancer drug therapy of ER- tumours.

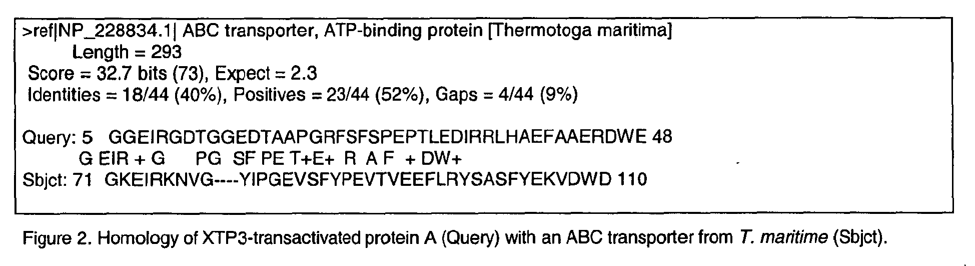

### Experiment 2: PR positive vs PR negative ER+ tumours

### Laser Capture PR positive versus PR negative tumours

In the above pilot study we established a sample pooling strategy and validated the results by comparison with known literature reports. We then applied a similar pooling regime to laser capture microdissected (LCM) tumour cells. In this case all tumours were ER+, but differed in being either positive (PR+) or negative (PR-) for the classical progesterone receptor (PR).

The estradiol effect requires the classical activation of target gene transcription by ERE (estrogen response element) binding, which is a prerequisite of the estrogen-dependent expression of the PR. Therefore, the presence of PR in tumor cells is used to reflect the functional integrity of the pathway, rather than simply the presence or absence of the receptor protein (Fuqua, 1996). Tumors expressing PR in addition to ER significantly and independently correlate with increased probability of response to tamoxifen, longer time to treatment failure, and longer overall survival (Ravdin et al., 1992; Mohsin et al., 2004). Response rates to tamoxifen are 43%, 53%, and 61 % in subsets of patients with less than 10, 10 to 99, and more than 100 fmol/mg PgR, respectively. In the ATAC trial, the largest trial to date comparing an aromatase inhibitor (arimidex) to tamoxifen it was revealed that ER+/PR- tumors are more responsive to Arimidex than ER+/PR+ lesions (Dowsett, 2003). Taken together, analysis of PR expression could help to separate patients with ER+ tumors who respond to estrogen/anti-estrogens from patients with ER+ tumors who do not or less thereby improving an individualized therapy strategy for patients.

In addition to clinical observations, several groups (Blobel et al., 1995; Philips et al., 1993; Stein and Yang, 1995; Umayahara et al., 1994) have demonstrated that in addition to the well known activation of target promoters containing EREs, other liganded ERα actions involving recruitment of ERα to gene promoters lacking an ERE via protein-protein interactions with promoter-bound transcription factors, or activation of the mitogen-activated protein kinase pathway, can take place. Based on this, we assumed that by comparing proteomic expression profiles of ER+ tumors with different expression of the PR proteins can be identified that are - unlike PR - regulated by an indirect non-genomic ERα action and might play a role in differential drug response. We therefore isolated homogeneous tumor cells from ER+/PR+ and ER+/PR- invasive ductal carcinoma using laser capture microdissection and analysed the protein lysates thereof using ProteoTope.

The design is shown in Table 5. Using the Arcturus LCM system, 10000 shots of 30 µm laser diameter were taken from each tumour. Sections were dehydrated as described (Wulfkuhle et al., 2002), stained with haemolysin/eosin, and cancer cells were specifically removed from surrounding stromal tissue. The microdissected tissue on caps was extracted directly into iodination buffer, and treated as above. Other experimental details follow experiment 1.

**Table 5. Pooling design for experiment 2. PR+ vs PR-, microdissected.**

| PR+ pools | T698 | T851 | T876 | T259 | T533 | T630 | T415 | T595 | T764 | T794 | T816 | T869 |
|---|---|---|---|---|---|---|---|---|---|---|---|---|
| PR+1 | + | + | + | | | | | | | | | |
| PR+2 | | | | + | + | + | | | | | | |
| PR+3 | | | | | | | + | + | + | | | |
| PR+4 | | | | | | | | | | + | + | + |
| | | | | | | | | | | | | |

| PR- pools | H623 | H69 | T680 | H579 | T382 | T894 | T425 | T413 | T802 | H574 | T2 | T3228 |
|---|---|---|---|---|---|---|---|---|---|---|---|---|
| PR-1 | + | + | + | | | | | | | | | |
| PR-2 | | | | + | + | + | | | | | | |
| PR-3 | | | | | | | + | + | + | | | |
| PR-4 | | | | | | | | | | + | + | + |

Individual tumours are designated by their tumour bank registration numbers.

The differential proteins consistently significantly detected between PR+ and PR- tumours is given in Table 6, and their mass spectrometric identification is documented in Table 7.

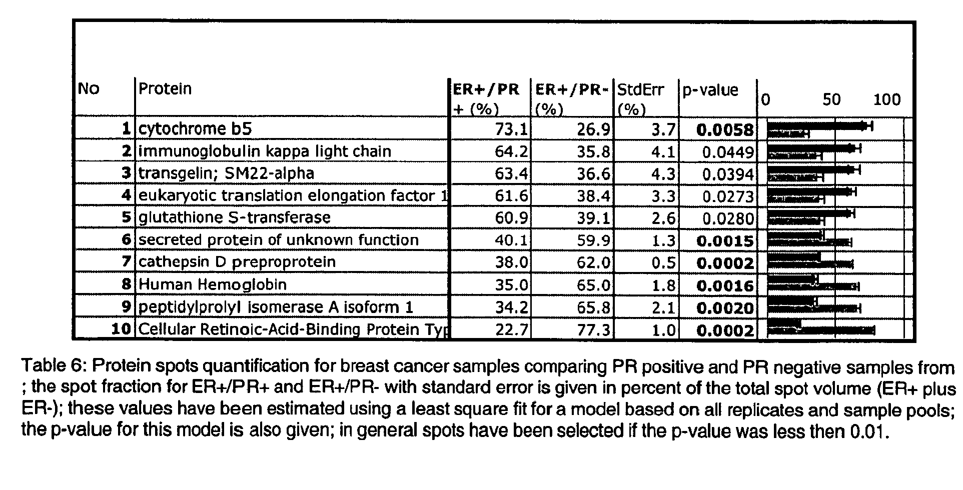

**Table 7: Mass spectrometric identification of all spots from ER+PR+ vs ER+PR- tumours that are congenic with spots from Table 6.**

| **No** | **exper.** | | **AccNo** | **Description** | **PMF Score** | **theor.** | |
|---|---|---|---|---|---|---|---|
| | **pl** | **MW** | | | | **pl** | **MW** |
| 1 | 4.7 | 16000 | gi\|353818 | cytochrome b5 | 102 | 4.8 | 11130 |
| 2 | 5.9 | 25000 | gi\|21669479 | immunoglobulin kappa light chain VLJ region [Homo sapiens] | 73 | 6.7 | 29807 |
| 3 | 6.4 | 18000 | gi\|4507359 | transgelin; SM22-alpha [Homo sapiens] | 113 | 8.9 | 22638 |
| 4 | 4.9 | 34000 | gi\|25453472 | eukaryotic translation elongation factor 1 delta isoform 2; guanine nucleotide exchange protein [Homo sapiens] | 88 | 4.6 | 31457 |
| 5 | 5.7 | 22000 | gi\|2204207 | glutathione S-transferase [Homo sapiens] | 84 | 5.3 | 24075 |
| 6 | 4.8 | 16000 | gi\|7019545 | secreted protein of unknown function [Homo sapiens] | 89 | 5.3 | 18845 |
| 7 | 5.3 | 28000 | gi\|4503143 | cathepsin D preproprotein [Homo sapiens] | 103 | 6.5 | 46117 |
| 8 | 7.0 | 12000 | gi\|1942686 | Chain B, Human Hemoglobin | 104 | 7.3 | 15988 |
| 9 | 7.0 | 15000 | gi\|10863927 | peptidylprolyl isomerase A isoform 1; cyclophilin A; peptidyl-prolyl cis-trans isomerase A; T cell cyclophilin; rotamase; cyclosporin A-binding protein [Homo sapiens] | 113 | 8.0 | 18709 |
| 10 | 5.4 | 13000 | gi\|3318697 | Chain A, Apo-Cellular Retinoic Acid Binding Protein II | 121 | 4.8 | 16058 |

One feature of Table 6 is that ER+PR+ resemble ER- tumours in having higher levels of immunoglobulin kappa light chain (lymphocyte infiltration), and lower Cathepsin D and hemoglobin (less vascularisation?) levels. Another point of note is that from the six proteins that exhibit significance < 0.001, retinoic acid binding protein type II is present with both cytochrome b5 and secreted protein of unknown function (SPUF). The latter are both cytochrome b5 domain-containing proteins, like Hpr6.6 that was identified as more abundant in ER- tumours above, and is thought to bind hormones via the cytochrome b5 domain (Mifsud and Bateman, 2002). Hormone binding of SPUF is likely to differentially modulate the biology of tumours of differing progesterone receptor status. One interesting suggestion is that cytochrome b5 itself, in addition to its heme ring-associated role in electron transport, may have a previously unrecognised role in hormone binding, however this interpretation requires further study. However SPUF represents a good candidate drug target for the treatment of cells negative for the PR.

Currently, progesterone receptor is a surrogate marker of estrogen receptor activity that is used to predict clinical outcome, most recently as assayed by immune histochemistry (Mohsin et al., 2004). The results of Table 6 indicate that the abundance of cytochrome b5 (higher abundance associated with significantly improved disease-free and overall survival) and cellular retinoic acid-binding protein type II (lower abundance associated with significantly improved disease-free and overall survival) offer extra diagnostic parameters to improve prediction of clinical outcome.

Furthermore, elevated abundance of the hormone-binding proteins SPUF and Hpr6.6 in tumours is associated with significantly worse disease-free and overall survival, offering extra diagnostic parameters to improve prediction of clinical outcome.

### References

Altschul SF, Madden TL, Schäffer AA, Zhang J, Zhang Z, Miller W, and Lipman DJ. 1997. Gapped BLAST and PSI-BLAST: a new generation of protein database search programs. Nucleic Acids Res. 25:3389-3402.
American Cancer Society. Cancer facts and figures, 2004. Blobel GA, Sieff CA, Orkin SH. 1995. Ligand-dependent repression of the erythroid transcription factor GATA-1 by the estrogen receptor. Mol Cell Biol 15:3147-3153.
Bowman CJ, Kroll KJ, Gross TG, Denslow ND. 2002. Estradiol-induced gene expression in largemouth bass, *Micropterus salmoides*. Mol Cell Endocrinol. 196:67-77.
Bray F, Sankila R, Ferlay J, and Parkin D, 2001. Estimates of cancer incidence and mortality in Europe in 1995, Eur J Cancer. 38:99-166.
Cahill MA, Wozny W, Schwall G, Schroer K, Holzer K, Poznanovic S, Hunzinger C, Vogt JA, Stegmann W, Matthies H, Schrattenholz A. (2003). Analysis of relative isotopologue abundances for quantitative profiling of complex protein mixtures labelled with the acrylamide/D3-acrylamide alkylation tag system. Rapid Communications in Mass Spectrometry, 2003, 17:1283-1290.
Chu PG, Weiss LM, 2002, Keratin expression in human tissues and neoplasms. Histopathology 40:403-439.
Come SE, Buzdar AU, Arteaga CL, Brodie AM, Davidson NE, Dowsett M, Ingle JN, Johnston SR, Lee AV, Osborne CK, Pritchard KI, Vogel VG, Winer EP, Hart CS. 2003. Second international conference on recent advances and future directions in endocrine manipulation of breast cancer: summary consensus statement. Clin Cancer Res 9:443-446.
Dowsett M. on behalf of the ATAC Trialist' Group. 2003. Analysis of time to recurrence in the ATAC (arimidex, tamoxifen, alone or in combination) trial according to estrogen receptor and progesterone receptor status. San Antonio Breast Cancer Symposium.
Fisher B, Jeong J, Dignam J, Anderson S, Mamounas E, Wickerham DL, and Wolmark N. 2001. Findings from recent National Surgical Adjuvant Breast and Bowel Project adjuvant studies in stage I breast cancer. J Natl Cancer Inst Monogr 30:62-66.
Fuqua, S. A. W. 1996. Estrogen and Progesterone Receptors and Breast Cancer. In: Diseases of the breast, edited by Harris, J. R., Lippman, M. E., Morrow, M. and Hellman, S. Lippincott-Raven, Philadelphia, New York.
Gerdes D, Wehling M, Leube B, Falkenstein E. 1998. Cloning and tissue expression of two putative steroid membrane receptors. Biol Chem. 379:907-11.
Gerner C, Steinkellner W, Holzmann K, Gsur A, Grimm R, Ensinger C, Obrist P, Sauermann G. 2001. Elevated plasma levels of crosslinked fibrinogen gamma-chain dimer indicate cancer-related fibrin deposition and fibrinolysis. Thromb Haemost. 85:494-50.
Gruvberger, S. et al. 2001. Estrogen receptor status in breast cancer is associated with remarkably distinct gene expression patterns. Cancer Res. 61, 5979-5984.
Hand RA, Craven RJ. 2003. Hpr6.6 protein mediates cell death from oxidative damage in MCF-7 human breast cancer cells. J Cell Biochem. 90:534-47.
Henderson IC. 1993. Risk factors for breast cancer development. Cancer 71:2127-2140.
Jemal A, Tiwari RC, Murray T, Ghafoor A, Samuels A, Ward E, Feuer EJ, Thun MJ; 2004. Cancer statistics, 2004. CA Cancer J Clin. 54:8-29.
Kendziorski CM, Zhang Y, Lan H, Attie AD. 2003. The efficiency of pooling mRNA in microarray experiments. Biostatistics. 4:465-77.
Lösel R, Christ M, Eisen C, Falkenstein E, Feuring M, Meyer C, Schultz A, Wehling M. 2003. Novel membrane-intrinsic receptors for progesterone and aldosterone. Chapter 15, pp 125-129 in: The identities of membrane steroid receptors. CS Watson editor. Kluwer Academic Publishers, Boston. ISBN 1-40207-344-5.
Mifsud W, Bateman A. 2002. Membrane-bound progesterone receptors contain a cytochrome b5-like ligand-binding domain. Genome-Biol. 3:RESEARCH0068.
Mohsin SK, Weiss H, Havighurst T, Clark GM, Berardo M, Roanh LD, To TV, Zho Q, Love RR, Allred DC. 2004. Progesterone receptor by immunohistochemistry and clinical outcome in breast cancer: a validation study. Mod Pathol. 2004 [Epub ahead of print]
Narod SA, Foulkes WD, 2004. BRCA1 and BRCA2: 1994 and beyond. Nature Reviews Cancer, 4:665.
Niveditha SR, Bajaj P. 2003. Vimentin expression in breast carcinomas. Indian J Pathol Microbiol. 46:579-584.
Osborne CK, 1998. Steroid hormone receptors in breast cancer management. Breast Cancer Res Treat. 51:227-238.
Pan W. 2002. A comparative review of statistical methods for discovering differentially expressed genes in replicated microarray experiments. Bioinformatics. 18:546-554.
Pastori RL, Moskaitis JE, Smith LH Jr, Schoenberg DR. 1990. Estrogen regulation of Xenopus laevis gamma-fibrinogen gene expression. Biochemistry. 29:2599-605.
Perou, C. M. et al. 2000. Molecular portraits of human breast tumours. Nature 406, 747-752.
Philips A, Chalbos D, Rochefort H. 1993. Estradiol increases and antiestrogens antagonize the growth factor-induced activator protein-1 activity in MCF7 breast cancer cells without affecting c-fos and c-jun synthesis. J Biol Chem 268:14103-14108.
Platet N, Prevostel C, Derocq D, Joubert D, Rochefort H, Garcia M. 1998. Breast cancer cell invasiveness: correlation with protein kinase C activity and differential regulation by phorbol ester in estrogen receptor-positive and -negative cells. Int J Cancer. 75:750-6.
Platet N, Cunat S, Chalbos D, Rochefort H, Garcia M. 2000. Unliganded and liganded estrogen receptors protect against cancer invasion via different mechanisms. Mol Endocrinol. 14:999-1009.
Platet N, Cathiard AM, Gleizes M, Garcia M. 2004. Estrogens and their receptors in breast cancer progression: a dual role in cancer proliferation and invasion. Crit Rev Oncol Hematol. 51:55-67.
Poland J, Cahill MA, Sinha P. 2003. Isoelectric focusing in long immobilized pH gradient gels to improve protein separation in proteomic analysis. Electrophoresis. 24:1271-1275.
Price JE, Polyzos A, Zhang RD, Daniels LM. 1990. Tumorigenicity and metastasis of human breast carcinoma cell lines in nude mice. Cancer Res 50:717-721.
Ravdin PM, Green S, Dorr TM, McGuire WL, Fabian C, Pugh RP, Carter RD, Rivkin SE, Borst JR, Belt RJ, et al. 1992. Prognostic significance of progesterone receptor levels in estrogen receptor-positive patients with metastatic breast cancer treated with tamoxifen: results of a prospective Southwest Oncology Group study. J Clin Oncol. 10:1284-91.
Rybarczyk BJ, Simpson-Haidaris PJ. 2000. Fibrinogen assembly, secretion, and deposition into extracellular matrix by MCF-7 human breast carcinoma cells. Cancer Res. 60:2033-2039.
Sauer, H. 2001. Mammakarzinome: Empfehlungen zur Diagnostik, Therapie und Nachsorge, 8^{th} edition. München: W. Zuckschwerdt Verlag.
Shevchenko A, Wilm M, Vorm O, Mann M. (1996). Mass spectrometric sequencing of proteins silver-stained polyacrylamide gels. Analytical Chemistry. 1996; 68:850-858.
Shiau AK, Barstad D, Loria PM, Cheng L, Kushner PJ, Agard DA, Greene GL. 1998. The structural basis of estrogen receptor/coactivator recognition and the antagonism of this interaction by tamoxifen. Cell. 95:927-37.
Sommer S, Hunzinger C, Schillo S, Klemm M, Biefang-Arndt K, Schwall G, Pütter, Hoelzer K, Schroer K, Stegmann W Schrattenholz A. 2004. Molecular analysis of homocysteic acid-induced neuronal stress. J. Proteome Res. 3:572-81.
Stein B, Yang MX. 1995. Repression of the interleukin-6 promoter by estrogen receptor is mediated by NF-κB and C/EBPβ. Mol Cell Biol 15:4971-4979.
Thompson EW, Paik S, Brunner N, Sommers CL, Zugmaier G, Clarke R, Shima TB, Torri J, Donahue S, Lippman ME, Martin GR, Dixon RB. 1992. Association of increased basement membrane invasiveness with absence of estrogen receptor and expression of vimentin in human breast cancer cell lines. J Cell Physiol 150:534-544.
Umayahara Y, Kawamori R, Watada H, Imano E, lwama N, Morishima T, Yamasaki Y, Kajimoto Y, Kamada T. 1994. Estrogen regulation of the insulin-like growth factor I gene transcription involves an AP-1 enhancer. J Biol Chem 269:16433-16442.
Van't Veer, L. J. et al. 2002. Gene expression profiling predicts clinical outcome of breast cancer. Nature 415, 530-536.
Vogt JA, Schroer K, Holzer K, Hunzinger C, Klemm M, Biefang-Arndt K, Schillo S, Cahill MA, Schrattenholz A, Matthies H, Stegmann W. 2003a. Protein abundance quantification in embryonic stem cells using incomplete metabolic labelling with 15N amino acids, matrix-assisted laser desorption/ionisation time-of-flight mass spectrometry, and analysis of relative isotopologue abundances of peptides. Rapid Communications in Mass Spectrometry, 2003;17:1273-1282.
Vuong GL, Weiss SM, Kammer W, Priemer M, Vingron M, Nordheim A, Cahill MA, (2000) Improved sensitivity proteomics by postharvest alkylation and radioactive labelling of proteins. Electrophoresis, 2000; 21:2594-2605.
Wangh LJ, Holland LJ, Spolski RJ, Aprison BS, Weisel JW. 1983. Xenopus fibrinogen. Characterization of subunits and hormonal regulation of biosynthesis. J Biol Chem. 258:4599-605.
Wulfkuhle JD, Sgroi DC, Krutzsch H, McLean K, McGarvey K, Knowlton M, Chen S, Shu H, Sahin A, Kurek R, Wallwiener D, Merino MJ, Petricoin EF 3rd, Zhao Y, Steeg PS. 2002. Proteomics of human breast ductal carcinoma in situ. Cancer Res. 62:6740-6749.

## Claims

1. Method for performing proteomics especially with human microdissected samples, **characterized in that** the samples are grouped into two or more pools based on parameters of interest, said pools revealing at least one protein that is differentially manifest between these pools but common to members of a given pool.

2. Method according to claim 1, **characterized in that** the human samples are pooled on the basis being positive or negative towards at least one receptor, particularly towards a hormone binding receptor.

3. Method according to claim 2, **characterized in that** the receptor is the estrogen receptor (ER) and/or the progesterone receptor (PR).

4. Method according to one of the preceding claims, **characterized in that** the protein is vimentin, particularly an isoform thereof being significantly expressed in breast cancer cells that are negative for the estrogen receptor (ER-), or that have substantially poorer disease-free and overall survival probability.

5. Method according to one of the preceding claims, **characterized in that** the protein is fibrinogen, particularly an isoform thereof, and/or fibrin being significantly expressed in cancer cells, especially breast cancer cells, that are negative for the estrogen receptor (ER-), or that have substantially poorer disease-free and overall survival probability.

6. Method according to one of the preceding claims, **characterized in that** the protein is the XTP3-transactivated protein A being significantly expressed in cancer cells, especially breast cancer cells that are negative for the estrogen receptor (ER-), or that have substantially poorer disease-free and overall survival probability.

7. Method according to one of the preceding claims, **characterized in that** the protein is the progesterone receptor membrane component 1 (Hpr6.6 protein), particularly two isoforms thereof being significantly expressed in cancer cells, especially breast cancer cells that are negative for the estrogen receptor (ER-), or that have substantially poorer disease-free and overall survival probability.

8. Method according to one of the preceding claims, **characterized in that** the protein is the retinoic acid-binding protein type II and/or secreted protein of unknown function (SPUF) being significantly expressed in cancer cells, especially breast cancer cells that are positive for the estrogen receptor (ER+) and negative for the progesterone receptor (PR-), or that have substantially poorer disease-free and overall survival probability.

9. Method according to one of the preceding claims, **characterized in that** the protein is cytochrome b5 being significantly expressed in cancer cells, especially breast cancer cells that are positive for the estrogen receptor (ER+) and positive for the progesterone receptor (PR+), or that have substantially better disease-free and overall survival probability.

10. Method according to one of the preceding claims, **characterized in that** the human sample is derived from a small tissue fraction, particularly from a tumour tissue fraction, advantageously from a breast cancer tissue fraction.

11. Use of a pooling strategy for performing proteomics especially with human microdissected samples, **characterized in that** the samples are grouped into two or more pools based on parameters of interest, said pools revealing at least one protein that is differentially manifest between these pools but common to members of a given pool.

12. Use of the isoform of vimentin whose expression is significantly increased in cancer cells, especially in breast cancer cells that are negative for the estrogen receptor (ER-), as a diagnostic marker for breast cancer patients that have substantially poorer disease-free and overall survival probability.

13. Use of fibrinogen, especially the isoforms thereof, and/or fibrin whose expression are significantly increased in cancer cells, especially breast cancer cells, especially in breast cancer cells that are negative for the estrogen receptor (ER-), as diagnostic markers for breast cancer patients that have substantially poorer disease-free and overall survival probability.

14. Use of the XTP3-transactivated protein A whose expression is significantly increased in cancer cells, especially breast cancer cells, especially in breast cancer cells that are negative for the estrogen receptor (ER-), as a diagnostic marker for breast cancer patients that have substantially poorer disease-free and overall survival probability.

15. Use of the cellular retinoic acid-binding protein type II whose expression is significantly increased in cancer cells, especially breast cancer cells, especially in breast cancer cells being positive for the estrogen receptor (ER+) and positive for the progesterone receptor (PR+), as diagnostic marker for breast cancer patients that have substantially better disease-free and overall survival probability.

16. Use of cytochrome b5 whose expression is significantly increased in cancer cells, especially breast cancer cells, especially in breast cancer cells being positive for the estrogen receptor (ER+) and negative for the progesterone receptor (PR-), as diagnostic marker for breast cancer patients that have substantially poorer disease-free and overall survival probability.

17. Use of secreted protein of unknown function (SPUF) whose expression is significantly increased in cancer cells, especially breast cancer cells, especially in breast cancer cells being positive for the estrogen receptor (ER+) and negative for the progesterone receptor (PR-), as diagnostic marker for breast cancer patients that have substantially poorer disease-free and overall survival probability.

18. Use of either one, or both, of two isoforms of progesterone receptor membrane component 1 (Hpr6.6 protein) whose expression are significantly increased in cancer cells, especially breast cancer cells, especially in breast cancer cells that are negative for the estrogen receptor (ER-), as diagnostic markers for breast cancer patients that have substantially poorer disease-free and overall survival probability.

19. Use of some or all of the relative protein abundances from Table 2 to diagnose breast cancer patients that have substantially poorer or better disease-free and overall survival probability.

20. Use of some or all of the relative protein abundances from Table 6 to diagnose breast cancer patients that have substantially poorer or better disease-free and overall survival probability.

21. Use of some or all of the relative protein abundances from Table 6 and Table 2 to diagnose breast cancer patients that have substantially poorer or better disease-free and overall survival probability.

22. Use of a reagent to modulate the activity or abundance of XTP3-transactivated protein A as a treatment for cancer cells, especially breast cancer cells, especially in breast cancer cells that are negative for the estrogen receptor (ER-), to improve patient disease-free and overall survival probability.

23. Use of a reagent to modulate the activity or abundance of cellular retinoic acid-binding protein type II as a treatment for cancer cells, especially breast cancer cells, especially in breast cancer cells being positive for the estrogen receptor (ER+) and positive for the progesterone receptor (PR+), to improve patient disease-free and overall survival probability.

24. Use of a reagent to modulate the activity or abundance of cytochrome b5 as a treatment for cancer cells, especially breast cancer cells, especially breast cancer cells being positive for the estrogen receptor (ER+) and negative for the progesterone receptor (PR-), to improve patient disease-free and overall survival probability.

25. Use of a reagent to modulate the activity or abundance of secreted protein of unknown function (SPUF) as a treatment for cancer cells, especially breast cancer cells, especially in breast cancer cells being positive for the estrogen receptor (ER+) and negative for the progesterone receptor (PR-), to improve patient disease-free and overall survival probability.

26. Use of a reagent to modulate the activity or abundance of progesterone receptor membrane component 1 (Hpr6.6 protein), or isoforms thereof, whose expression are significantly increased in cancer cells, especially breast cancer cells, especially in breast cancer cells that are negative for the estrogen receptor (ER-), to improve patient disease-free and overall survival probability.
